# EUROPEAN PATENT SPECIFICATION

(11) **EP 1 015 035 B1**
(45) Date of publication and mention of the grant of the patent: **28.01.2009**
(21) Application number: 98939363.2
(22) Date of filing: 13.08.1998
(51) Int. Cl.: A61K 48/00, C12N 5/10, C12N 15/09, C12N 15/63, A61K 39/00

(54) **VACCINATION BY TOPICAL APPLICATION OF GENETIC VECTORS**
IMPFUNG DURCH TOPISCHE VERWENDUNG GENETISCHER VEKTOREN
VACCINATION PAR APPLICATION TOPIQUE DE VECTEURS GENETIQUES

(30) Priority: 13.08.1997 US 55520 P; 11.02.1998 US 75113 P
(43) Date of publication of application: 05.07.2000
(73) Proprietor: THE UAB RESEARCH FOUNDATION, Birmingham, AL 35294-0111 (US)
(72) Inventor: TANG, De-chu, Christopher, Birmingham, AL 35244 (US); MARKS, Donald, H., Rockaway, NJ 07866 (US); CURIEL, David, T., Birmingham, AL 35222 (US); SHI, Zhongkai, Birmingham, AL 35205 (US)
(74) Representative: Plougmann & Vingtoft A/S
(86) International application number: PCT/US1998/016739
(87) International publication number: WO 1999/008713

(56) References cited:
- WO-A-96/14074
- WO-A-98/03641
- WO-A-99/41366
- US-A- 3 837 340
- US-A- 5 679 647
- TANG ET AL: "Vaccination onto bare skin" NATURE, MACMILLAN JOURNALS LTD. LONDON, GB, vol. 388, no. 6644, 21 August 1997 (1997-08-21), pages 729-730, XP002110051 ISSN: 0028-0836
- LU B ET AL: "TOPICAL APPLICATION OF VIRAL VECTORS FOR EPIDERMAL GENE TRANSFER" JOURNAL OF INVESTIGATIVE DERMATOLOGY, NEW YORK, NY, US, vol. 108, no. 5, May 1997 (1997-05), pages 803-808, XP002913861 ISSN: 0022-202X
- DATABASE BIOSIS [Online] BIOSCIENCES INFORMATION SERVICE, PHILADELPHIA, PA, US; 1994, FULLER DEBORAH HEYDENBURG ET AL: "A qualitative progression in HIV type 1 glycoprotein 120-specific cytotoxic cellular and humoral immune responses in mice receiving a DNA-based glycoprotein 120 vaccine" XP002305513 Database accession no. PREV199598082543 & AIDS RESEARCH AND HUMAN RETROVIRUSES, vol. 10, no. 11, 1994, pages 1433-1441, ISSN: 0889-2229
- DATABASE EMBASE [Online] ELSEVIER SCIENCE PUBLISHERS, AMSTERDAM, NL; 1994, HAYNES J R ET AL: "Accell(R) particle-mediated DNA immunization elicits humoral, cytotoxic, and protective immune responses" XP002305514 Database accession no. EMB-1994369175 & AIDS RESEARCH AND HUMAN RETROVIRUSES 1994 UNITED STATES, vol. 10, no. SUPPL. 2, 1994, pages S43-S45, ISSN: 0889-2229
- DATABASE BIOSIS [Online] BIOSCIENCES INFORMATION SERVICE, PHILADELPHIA, PA, US; 1993, FYNAN ELLEN F ET AL: "DNA vaccines: Protective immunizations by parenteral, mucosal, and gene-gun inoculations" XP002305515 Database accession no. PREV199497089719 & PROCEEDINGS OF THE NATIONAL ACADEMY OF SCIENCES OF THE UNITED STATES OF AMERICA, vol. 90, no. 24, 1993, pages 11478-11482, ISSN: 0027-8424
- NIEMIEC SUSAN M ET AL: "Perifollicular transgenic expression of human interleukin-1 receptor antagonist protein following topical application of novel liposome-plasmid DNA formulations in vivo" JOURNAL OF PHARMACEUTICAL SCIENCES, vol. 86, no. 6, 1997, pages 701-708, XP002913862 ISSN: 0022-3549
- DATABASE EMBASE [Online] ELSEVIER SCIENCE PUBLISHERS, AMSTERDAM, NL; 1995, PAUL A ET AL: "Noninvasive administration of protein antigens: Transdermal immunization with bovine serum albumin in transfersomes" XP002305516 Database accession no. EMB-1995351650 & VACCINE RESEARCH 1995 UNITED STATES, vol. 4, no. 3, 1995, pages 145-164, ISSN: 1056-7909
- LI L ET AL: "The feasibility of targeted selective gene therapy of the hair follicle" NATURE MEDICINE, NATURE PUBLISHING, CO, US, vol. 1, no. 7, July 1995 (1995-07), pages 705-706, XP002140346 ISSN: 1078-8956
- SHI Z ET AL: "DNA-based non-invasive vaccination onto the skin" VACCINE, BUTTERWORTH SCIENTIFIC. GUILDFORD, GB, vol. 17, no. 17, 23 April 1999 (1999-04-23), pages 2136-2141, XP004164998 ISSN: 0264-410X
- FAN H ET AL: "IMMUNIZATION VIA HAIR FOLLICLES BY TOPICAL APPLICATION OF NAKED DNATO NORMAL SKIN" NATURE BIOTECHNOLOGY, NATURE PUBLISHING, US, vol. 17, September 1999 (1999-09), pages 870-872, XP002929843 ISSN: 1087-0156
- YOKOYAMA et al., "DNA Immunization: Effects of Vehicle and Route of Administration on the Induction of Protective Antiviral Immunity", FEMS IMMUNOLOGY AND MEDICAL MICROBIOLOGY, 1996, Vol. 14, No. 4, pages 221-230, XP002913860
- LU et al., "Topical Application of Viral Vectors for Epidermal Gene Transfer", JOURNAL OF INVESTIGATIVE DERMATOLOGY, May 1997, Vol. 108, No. 5, pages 803-808, XP002913861
- NIEMIEC et al., "Perifollicular Transgenic Expression of Human Interleukin-1 Receptor Antagonist Protein Following Topical Application of Novel Liposome-Plasmid DNA Formulations In Vivo", JOURNAL OF PHARMACEUTICAL SCIENCES, June 1997, Vol. 86, No. 6, pages 701-708, XP002913862
- WEINER N., "Targeted Follicular Delivery of Macromolecules via Liposomes", INTERNATIONAL J. OF PHARMACEUTICS, January 1998, Vol. 162, No. 1-2, pages 29-38, XP002913863

## Description

### Background of the Invention

### Fiel of the Invention

The present invention relates generally to the fields of immunology and vaccine technology. More specifically, the present invention relates to techniques of skin-targeted non-invasive gene delivery to elicit immune responses and uses thereof.

### Description of the Related Art

Activation of the immune system of vertebrates is an important mechanism for protecting animals against pathogens and malignant tumors. The immune system consists of many interacting components including the humoral and cellular branches. Humoral immunity involves antibodies that directly bind to antigens. Antibody molecules as the effectors of humoral immunity are secreted by B lymphocytes. Cellular immunity involves specialized cytotoxic T lymphocytes (CTLs) which recognize and kill other cells which produce non-self antigens. CTLs respond to degraded peptide fragments that appear on the surface of the target cell bound to MHC (major histocompatibility complex) class I molecules. It is understood that proteins produced within the cell are continually degraded to peptides as part of cellular metabolism. These fragments are bound to the MHC molecules and are transported to the cell surface. Thus the cellular immune system is constantly monitoring the spectra of proteins produced in all cells in the body and is poised to eliminate any cells producing non-self antigens.

Vaccination is the process of priming an animal for responding to an antigen. The antigen can be administered as a protein (classical) or as a gene which then expresses the antigen (genetic immunization). The process involves T and B lymphocytes, other types of lymphoid cells, as well as specialized antigen presenting cells (APCs) which can process the antigen and display it in a form which can activate the immune system. Current modes for the administration of genetic vaccines has focused on invasive procedures including needle injections, scarification, and gene gun-mediated penetration. Inoculation of vaccines in an invasive mode requires equipment and personnel with special medical training, and is usually associated with discomfort and potential hazards (bleeding, infection). There is now evidence that the inoculation of vaccines in an invasive mode may be unnecessary (Tang et al., 1997; Glenn et al., 1998). In WO 96/14074, plasmid encoding antigen may be delivered to the skin; serum antibodies can be generated against the encoded antigen. Since the skin interfaces directly with the external environment and is in constant contact with potential pathogens, the immune system must constantly keep a mobilized biological army along the skin border for warding off potential infections. As a consequence, the outer layer of skin is essentially an immunocompetent tissue. Immunologic components present in the skin for the elicitation of both humoral and cytotoxic cellular immune responses include epidermal Langerhans cells (which are MHC class II-positive antigen-presenting cells), keratinocytes, and both CD4⁺ and CD8⁺ T lymphocytes. These components make the skin an ideal site for administration of vaccine. The large accessible area of skin and its durability are other advantages for applying vaccines to this tissue. Expression of a small number of antigens in the outer layer of skin without physical penetration may thus elicit a potent immune response by alarming the immune surveillance mechanism.

The efficacy of a vaccine is measured by the extent of protection against a later challenge by a tumor or a pathogen. Effective vaccines are immunogens that can induce high titer and long-lasting immunity for targeted intervention against diseases after a minimum number of inoculations. For example, genetic immunization is an approach to elicit immune responses against specific proteins by expressing genes encoding the proteins in an animal's own cells. The substantial antigen amplification and immune stimulation resulting from prolonged antigen presentation *in* vivo can induce a solid immunity against the antigen. Genetic immunization simplifies the vaccination protocol to produce immune responses against particular proteins because the often difficult steps of protein purification and combination with adjuvant, both routinely required for vaccine development, are eliminated. Since genetic immunization does not require the isolation of proteins, it is especially valuable for proteins that may lose conformational epitopes when purified biochemically. Genetic vaccines may also be delivered in combination without eliciting interference or affecting efficacy (Tang et al., 1992; Barry et al., 1995), which may simplify the vaccination scheme against multiple antigens. It has been demonstrated, as presented in this application, that genetic vaccines can be inoculated in a novel way as skin-targeted non-invasive vaccines. The combination of genetic vaccines with a non-invasive delivery mode may result in a new class of "democratic" vaccines that require no special skill and equipment for administration.

While topically-applied protein-based vaccines have been studied, their usefulness may be limited. Although topical application of protein-based vaccines in conjunction with cholera toxin may also immunize animals in the same non-invasive mode (Glenn et al., 1998) as skin-targeted non-invasive genetic vaccines have already been shown to do (Tang et al., 1997), the two classes of vaccines activate the immune system via different mechanisms. Further, the efficacy of genetic vaccines is in general superior to that of protein vaccines due to the *de novo* synthesis of antigens similar to natural infections (McDonnell and Askari, 1996). Although U.S. Pat. No. 3,837,340 describes a method for vaccinating animals by contacting skin with dried viruses, the viruses that they employ are not genetic vectors capable of expressing transgenes. In addition, the immunogen may be protein in the viral coat, instead of protein produced from expression of viral genes in animals' own cells.

The prior art of vaccination usually requires equipment, e.g., syringe needles or a gene gun, and special skill for the administration of vaccines. There is a great need and desire in the art for the inoculation of vaccines by personnel without medical training and equipment. A large number of diseases could potentially be immunized against through the development of non-invasive vaccination onto the skin (NIVS) because the procedure is simple, effective, economical, painless, and potentially safe. As a consequence, NIVS may boost vaccine coverages in developing countries where medical resources are in short supply, as well as in developed countries due to patient comfort. Infectious diseases caused by viruses, including AIDS and flu, by bacteria, including tetanus and TB, and by parasites, including malaria, and malignant tumors including a wide variety of cancer types may all be prevented or treated with skin-targeted non-invasive vaccines without requiring special equipment and medical personnel. The present invention satisfies this longstanding need and desire in the art.

### Summary of the Invention

Non-invasive vaccination onto the skin (NIVS) can improve vaccination schemes because skin is an immunocompetent tissue and this non-invasive procedure requires no specially trained personnel. Skin-targeted non-invasive gene delivery can achieve localized transgene expression in the skin and the elicitation of immune responses (Tang et al., 1997). These results indicate that NIVS is a-novel and efficient method for the delivery of vaccines. The simple, effective, economical and painless immunization protocol of the present invention should make vaccination less dependent upon medical resources and, therefore, increase the annual utilization rate of vaccinations.

The present invention thus relates to use of an adenoviral vector encoding a transgene of interest for the preparation of a non-invasive vaccine for the induction of an immune response in an animal by non-invasive application of the vector to the skin of the animal.

### Brief Description of the Drawings

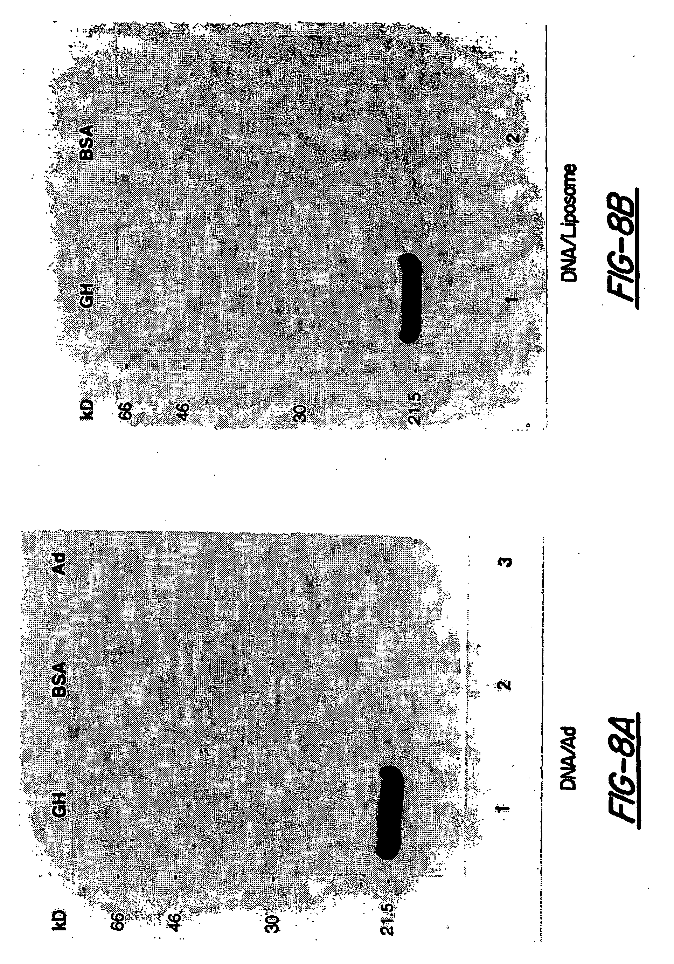

So that the matter in which the above-recited features, advantages and objects of the invention, as well as others which will become clear, are attained and can be understood in detail, more particular descriptions of the invention briefly summarized above may be had by reference to certain embodiments thereof which are illustrated in the appended drawings. These drawings form a part of the specification. It is to be noted, however, that the appended drawings illustrate preferred embodiments of the invention and therefore are not to be considered limiting in their scope. Figure 8b does not illustrate the invention.
Figure 1 shows the transgene expression from adenovirus recombinants in the skin by topical application of the vectors;
Figures 2a and 2b show the characterization of potential target cells that can be transduced by topically-applied adenovirus recombinants;
Figures 3a and 3b show the detection of specific antibodies in the sera of mice immunized by adenovirus-mediated NIVS;
Figure 4 shows the percent survival of control versus immunized mice that were challenged by a lethal dose of tumor cells;
Figure 5 shows the characterization of tumor-infiltrating T lymphocytes;
Figure 6 shows the characterization of tumor-infiltrating CTLs;
Figure 7 shows the western blot analysis of antibodies to the human CEA protein in mice immunized by topical application of vaccine bandages;
Figure 8a shows the detection of specific antibodies in the serum of a mouse immunized by DNA/adenovirus-mediated NIVS;
Figure 8b shows the detection of specific antibodies in the serum of a mouse immunized by DNA/liposome-mediated NIVS;
Figure 9 shows the co-expression of DNA-encoded and adenovirus-encoded transgenes in target cells;
Figure 10 shows relative transgene expression from topically-applied adenovirus recombinants, DNA/adenovirus complexes, and DNA/liposome complexes;
Figure 11 shows a device for the administration of skin-targeted non-invasive vaccines.

### Detailed Description of the Invention

The present invention relates to use of an adenoviral vector encoding a transgene of interest for the preparation of a non-invasive vaccine for the induction of an immune response in an animal by non-invasive application of the vector to the skin of the animal.

In one embodiment according to the present invention, said transgene encodes an anti tumor antigen and said immune response is an anti tumor immune response.

In a second embodiment according to the present invetnion, the transgene encodes an antigen.

In a third embodiment according to the present invention, the antigen can be used against a pathogen or neoplasm.

In a fourth embodiment according to the present invention, the antigen is selected from the group consisting essentially of the human carcinoembryonic antigen, the HIV gp120 antigen, and the influenza NP antigens.

In a fifth embodiment according to the present invention, the vector further encodes a co-stimulatory gene and a cytokine gene.

In a sixth embodiment according to the present invention, the immune modulatory gene is selected from the group consisting of a GM-CSF gene, a B7-1 gene, a B7-2 gene, an interleukin-2 gene, and an interleukin-12 gene.

In a seventh embodiment according to the present invention, the adenovirus vector is defective in its E1 region.

In an eighth embodiment according to the present invention, the vector has all viral genes deleted.

In a final embodiment according to the present invention, the vector comprises a DNA/virus complex and the DNA is in plasmid form.

Representative examples of antigens which can be used to produce an immune response include the human carcinoembryonic antigen, the HIV gp120, the tetanus toxin C-fragment, and the influenza HA and NP, etc. Most preferably, the immune response produces a protective effect against neoplasms or infectious pathogens.

Genetic vectors operatively coding for a polypeptide may be delivered into the outer layer of skin of a vertebrate by a non-invasive procedure for immunizing the animal. These genetic vectors can be administered to the vertebrate by direct transfer of the genetic material to the skin without utilizing any devices, or by contacting naked skin utilizing a bandage or a bandage-like device. In preferred applications, the genetic vector is in aqueous solution. Vectors reconstituted from lyophilized powder are also acceptable. The vector may encode a complete gene, a fragment of a gene or several genes, gene fragments fused with immune modulatory sequences such as ubiquitin or CpG-rich synthetic DNA, together with transcription/translation signals necessary for expression.

The vector further contains a vector may furthermore contain a gene selected from the group consisting of co-stimulatory genes and cytokine genes. The gene is the gene may be selected from the group consisting of a GM-CSF gene, a B7-1 gene, a B7-2 gene, an interleukin-2 gene, an interleukin-12 gene and interferon genes.

In the embodiments of the invention that require use of adenovirus recombinants, it may include E1-defective adenovirus vectors, or the "gutless" adenovirus vector in which all viral genes are deleted. The E1 mutation raises the safety margin of the vector because E1-defective adenovirus mutants are replication incompetent in non-permissive cells. The "gutless" adenovirus vector is the latest model in the adenovirus vector family. Its replication requires a helper virus an a special human 293 cell line expressing both Ela and Cre, a condition that does not exist in natural environment; the vector is deprived of all viral genes, thus the vector as a vaccine carrier is non-immunogenic and may be inoculated for multiple times for revaccination. The "gutless" adenovirus vector also contains 36 kb space for accommodating transgenes, thus allowing co-delivery of a large number of antigen genes into cells. Specific sequence motifs such as the RGD motif may be inserted into the H-I loop of an adenovirus vector to enhance its infectivity. An adenovirus recombinant is constructed by cloning specific transgenes or fragments of transgenes into any of the adenovirus vectors such as those described above. The adenovirus recombinant is used to transduce epidermal cells of a vertebrate in a non-invasive mode for use as an immunizing agent.

In the embodiments of the invention that require use of DNA/adenovirus complexes, it requires plasmid DNA complexed with adenovirus vectors utilizing either PEI (polyethylenimine) or polylysine. The adenovirus vector within the complex may be either "live" or "killed" by UV irradiation. The UV-inactivated adenovirus vector as a receptor-binding ligand and an endosomolysis agent for facilitating DNA-mediated transfection (Cotten et al., 1992) may raise the safety margin of the vaccine carrier. The DNA/adenovirus complex is used to transfect epidermal cells of a vertebrate in a non-invasive mode for use as an immunizing agent.

Genetic vectors provided by the invention can also code for immune modulatory molecules which can act as an adjuvant to provoke a humoral and/or cellular immune response. Such molecules include cytokines, co-stimulatory molecules, or any molecules that may change the course of an immune response. One can conceive of ways in which this technology can be modified to enhance still further the immunogenicity of antigens.

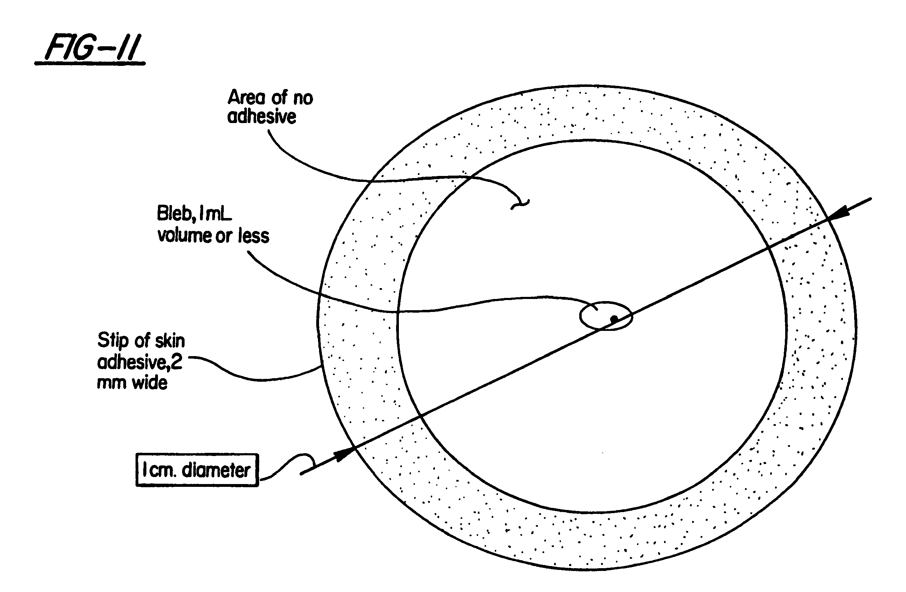

The genes can be delivered by various methods including device-free topical application or coating the genes on the surface of the skin of an animal by a device such as a pad or bandage; e.g., an adhesive bandage. Referring to Figure 11, a device for non-invasive vaccination is shown. This vaccine delivery device includes a non-allergenic, skin adhesive patch having a bleb disposed therein. In one embodiment, the patch is further comprised of plastic, approximately 1 cm in diameter. The vaccine can be disposed within the bleb. In another embodiment, the bleb contains approximately 1 mL of vaccine (as liquid, lyophilized powder with reconstituting fluid, and variants thereof). In a preferred embodiment, the surface of the bleb in contact with the skin is intentionally weaker than the opposite surface, such that when pressure is applied to the opposite surface, the lower surface breaks and releases the vaccine contents of the bleb onto the skin. The plastic patch traps the vaccine against the skin surface.

Dosage forms for the topical administration of the genetic vector and gene of interest of this invention can include liquids, ointments, powders, and sprays. The active component can be admixed under sterile conditions with a physiologically acceptable carrier and any preservatives, buffers, propellants, or absorption enhancers as may be required.

In terms of the terminology used herein, an immunologically effective amount is an amount or concentration of the genetic vector encoding the gene of interest, that, when administered to an animal, produces an immune response to the gene product of interest.

Various antigens may be delivered topically at different concentrations. Generally, useful amounts for adenovirus vectors are at least approximately 100 pfu and for plasmid DNA at least approximately 1 ng of DNA.

The invention can be appropriately applied to prevent diseases as prophylactic vaccination or treat diseases as therapeutic vaccination.

The vaccines prepared by the use of the present invention can be administered to an animal either alone or as part of an immunological composition.

Beyond the human vaccines described, the invention can be employed to immunize animal stocks. The term animal means all animals including humans. Examples of animals include humans, cows, dogs, cats, goats, sheep, and pigs, etc. Since the immune systems of all vertebrates operate similarly, the applications described can be implemented in all vertebrate systems.

The following examples (except example 10) are given for the purpose of illustrating various embodiments of the invention and are not meant to limit the present invention in any fashion.

### Protocols

### Mice and cell cultures

Inbred mice were maintained at the University of Alabama at Birmingham. Cells were cultured in RPMI 1640 or DMEM media containing 2% fetal bovine serum and 6% calf serum.

### Topical application of genetic vectors

Mice were anesthetized and hair and cornified epithelium covering a restricted area of abdominal or neck skin were removed by a depilatory (e.g., NAIR). Genetic vectors were pipetted onto the preshaved and NAIR-treated skin and kept in contact with naked skin for varying amounts of time (e.g., 1 hour to 18 hours). Vectors may be pipetted directly onto naked skin, or into a cylinder that is glued onto the skin.

### Preparation of adenovirus vectors

High titer adenovirus stocks were prepared from human 293 cells infected with specific adenovirus recombinants. Lysates were subjected to ultracentrifugation through a cesium chloride gradient. Viral bands were extracted and dialyzed against 10 mM Tris (pH 7.5)/135 mM NaCl/5 mM KCl/l mM MgCl₂. Purified viruses were filter sterilized with glycerol added to 10%, and stored in aliquots at -80°C. Titer for adenovirus stocks was determined by plaque assay.

### Luciferase assay

The amount of luciferase in the skin was determined as previously described (Tang, 1994). Briefly, a piece of excised skin was homogenized with a Kontes glass tissue grinder in lysis buffer. After removing tissue debris by centrifugation, luciferase activity in the skin extract was determined with a luminometer by measurement of integrated light emission in the presence of excess ATP and luciferin.

### β-Galactosidase assay

A piece of excised skin was quickly frozen in Tissue-Tek O.C.T. compound (Miles Laboratories Inc.) in liquid nitrogen and stored at -80°C until use. The frozen tissue was cross sectioned at 4 µm, fixed in 4% paraformaldehyde, and stained for β-galactosidase activity by incubation in X-gal staining solution as previously described (Tang et al., 1994). Sections were counterstained with haematoxylin and eosin.

### Preparation of DNA/adenovirus complexes

DNA/adenovirus complexes were prepared by mixing 100 µg plasmid DNA with 1x10¹¹ particles of adenovirus in the presence of the condensing agent polylysine for each application. The titer of adenovirus was determined by absorbance.

### Preparation of DNA/liposome complexes

DNA/liposome complexes were prepared by mixing 100 µg plasmid DNA with 100 µg DOTAP/DOPE (1:1; Avanti) for each application. Plasmids were prepared using Qiagen Plasmid Maxi Kits.

### Western blot analysis

Sera from tail bleeds were diluted 1:250 to 1:500 and reacted with purified proteins that had been separated in a SDS-polyacrylamide gel and transferred to an Immobilon-P membrane (Millipore). Reaction was visualized using the ECL kit (Amersham).

### EXAMPLE 1

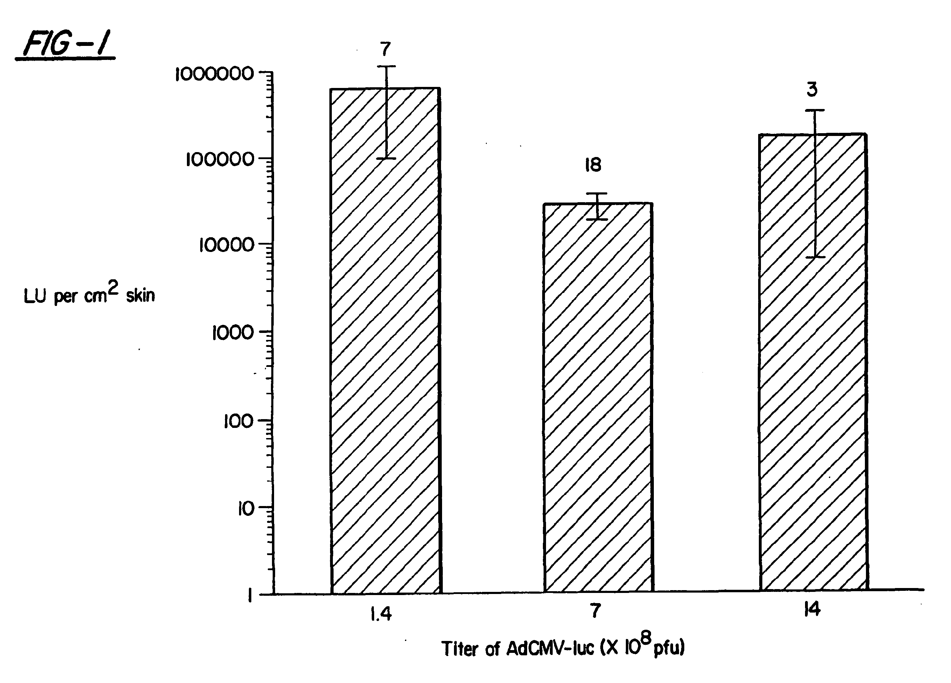

The present invention demonstrates that antigen genes can be delivered into the skin of mice in a simplified manner by skin-targeted non-invasive delivery of a genetic vector without using sophisticated equipment. Figure 1 shows that substantial amounts of luciferase enzyme was produced after delivery of limited amounts of AdCMV-luc (an adenovirus vector encoding the firefly luciferase) (Tang et al., 1994) onto the skin. Ad, adenovirus; pfu, plaque-forming units; LU, light units. Results are the mean log[LU per cm² skin] ± SE (n is shown on top of each column). Mice mock-applied or coated with an adenovirus vector that did not encode luciferase produced no detectable luciferase activity in the skin. The level of transgene expression from the adenovirus vector in the skin did not appear to correlate with the titer of the virus. It is possible that only a small number of cells can be transduced by the virus in a restricted subset of skin, and 10⁸ plaque-forming units (pfu) of adenovirus recombinants may have saturated the target cells. This variability could also be due, in part, to variations of individual mice. In addition, some of the variability probably arose from the procedure for removing cornified epithelium which had not been standardized (Johnston and Tang, 1994). The amount of antigen produced may potentially be amplified by applying more vectors onto a larger area.

### EXAMPLE 2

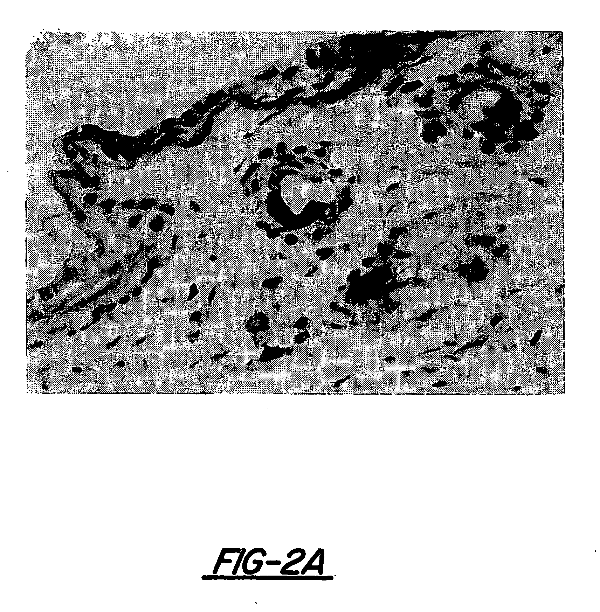

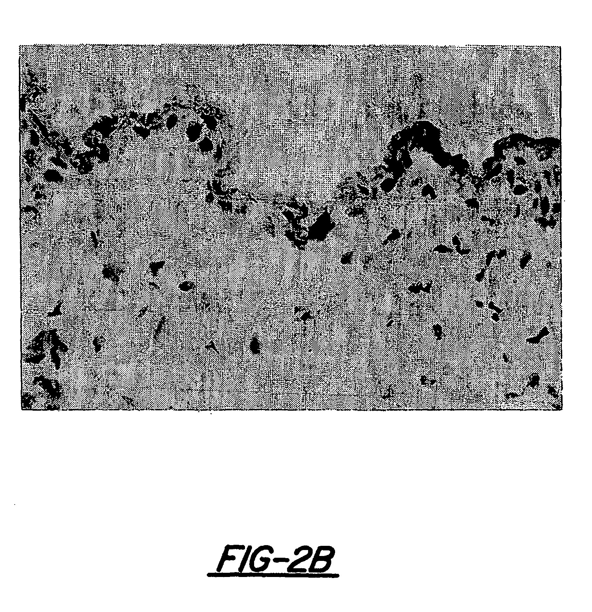

The principal target cells for non-invasive vaccination onto the skin appeared to be hair matrix cells within hair follicles (Figure 2a) and keratinocytes within the outermost layer of epidermis (Figure 2b) as shown by staining frozen sections with X-gal substrates after skin-targeted non-invasive delivery of an adenovirus vector encoding the *E*. *coli* β-galactosidase gene (AdCMV-βgal) (Tang et al., 1994). No physical abrasions were found in the skin tissue subjected to the treatment, and there was no inflammation induced. The skin tissue subjected to non-invasive gene delivery was excised from animals 1 day after pipetting 10⁸ pfu of AdCMV-βgal onto the skin, cross sectioned, fixed, and stained with X-gal substrates as described (Tang et al., 1994). Figure 2a shows the adenovirus-transduced hair matrix cells within a hair follicle, x 150. Figure 2b shows the adenovirus-transduced keratinocytes within the outermost layer of epidermis, x 150. No blue cells were found in control animals that were either mock-applied or coated with AdCMV-luc.

### EXAMPLE 3

### Elicitation of humoral immune responses by adenovirus-mediated NIVS

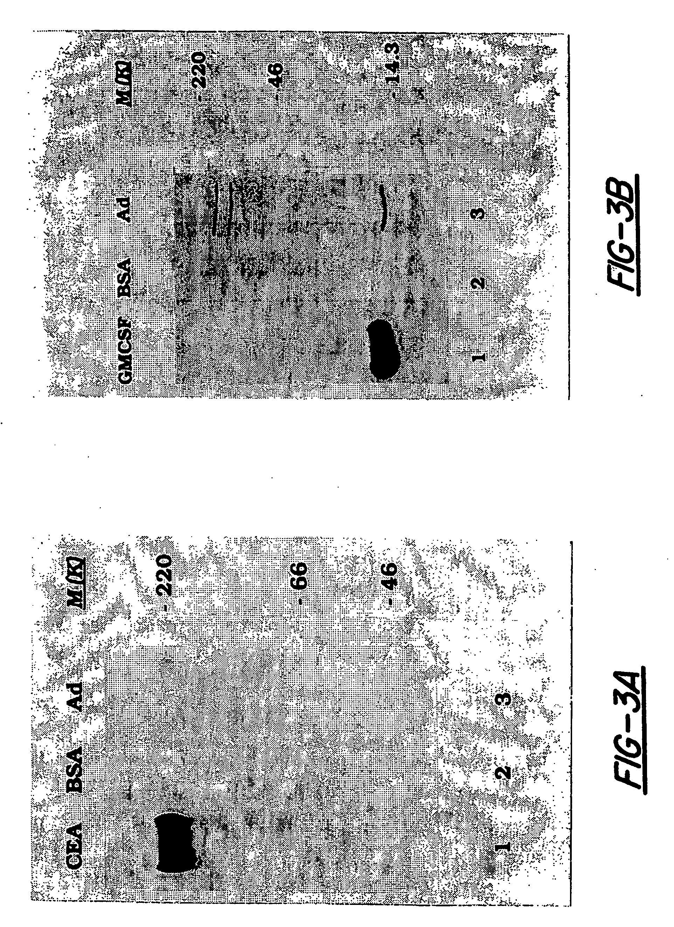

NIVS is a novel method for vaccinating animals. To demonstrate that the procedure can elicit a specific immune response against the antigen encoded by the vector, AdCMV-hcea [an adenovirus vector encoding the human carcinoembryonic antigen (CEA)] was pipetted onto the skin of the C57BL/6 strain mice. Serum from a vaccinated mouse a month after skin-targeted non-invasive delivery of 10⁸ pfu AdCMV-hcea was diluted 1:500 and reacted with purified human CEA protein (provided by T. Strong) and adenoviral proteins that had been separated in a 5% SDS-polyacrylamide gel, and transferred to Immobilon-P membranes (Millipore). Referring to Figure 3a, lane 1, 0.5 µg of human CEA; lane 2, 0.5 µg of BSA; lane 3, 10⁷ pfu of adenovirus. Figure 3a shows that the test sera from a vaccinated animal reacted in western blots with purified human CEA protein, but not with bovine serum albumin (BSA), which supports the conclusion that specific antibodies have been produced against exogenous proteins encoded by adenovirus vectors as a result of skin-targeted non-invasive gene delivery.

To test whether this technique might be generally applicable, AdCMV-hgmcsf [an adenovirus vector encoding the human granulocyte macrophage colony stimulating factor (hGM-CSF)] was applied onto the skin. To detect antibodies against the human GM-CSF protein, the animal was vaccinated by skin-targeted non-invasive delivery of 10⁸ pfu of AdCMV-hgmcsf. Purified human GM-CSF protein (CalBiochem) separated in a 15% SDS-polyacrylamide gel was transferred to membranes and allowed to react with diluted serum. Other treatments were carried out as described in Figure 3a. Referring to Figure 3b, lane 1, 0.25 µg of human GM-CSF; lane 2, 0.25 µg of BSA; lane 3, 10⁷ pfu of adenovirus. The replication-defective human adenovirus serotype 5 derived AdCMV-hcea and AdCMV-hgmcsf were produced in human 293 cells. A cassette containing the human CEA gene or the human GM-CSF gene, driven by the cytomegalovirus (CMV) early enhancer-promoter element was inserted in place of the E1a deletion. Since the sequences in the E1a region were deleted, the ability of these viruses to replicate autonomously in nonpermissive cells was impaired.

Results (Tang et al., 1997) show that 96% (23/24) of the C57BL/6 strain mice produced antibodies against the human CEA protein a month after skin-targeted non-invasive delivery of AdCMV-hcea, and 43% (6/14) of the same strain mice produced antibodies against the human GM-CSF protein after skin-targeted non-invasive delivery of AdCMV-hgmcsf. Both pre-immune sera collected before NIVS and sera from naive animals failed to react with the human CEA and GM-CSF proteins. The possibility of oral vaccination by ingesting vectors through grooming was eliminated by (1) rinsing vectors away from the skin before animals recovered from anesthesia, (2) pipetting vectors onto unshaved skin, and (3) mixing naive and vaccinated animals in the same cage. No cross-vaccination between naive and vaccinated mice was ever observed, and shaving appeared as an essential component for NIVS presumably due to the mechanical removal of cornified epithelium along the shaving path. Thus, adenovirus-mediated NIVS is capable of eliciting a humoral immune response against an antigen encoded by the vector.

### EXAMPLE 4

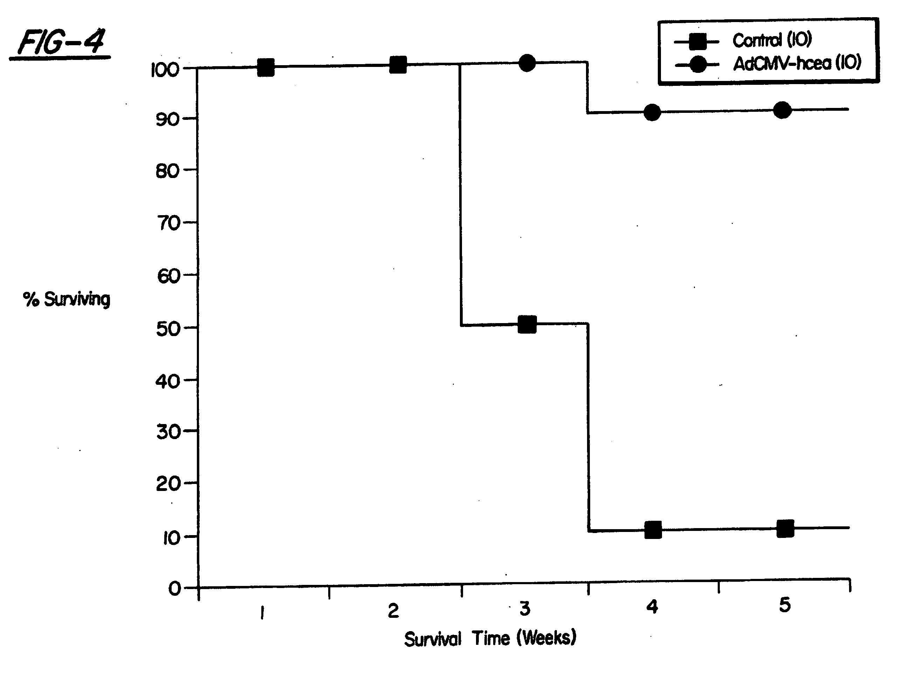

To demonstrate that the techniques of the present invention can elicit a protective antitumor immune response, syngeneic tumor cells that express the human carcinoembryonic antigen (CEA) gene (MC38-CEA-2) (Conry et al., 1995) were inoculated into naive C57BL/6 strain mice and the same strain mice that had been vaccinated by topical application of an adenovirus vector encoding the human CEA gene (AdCMV-hcea). Animals subjected to tumor challenges were observed for survival (Figure 4). In the control group, 90% (9/10) of the animals developed palpable tumor nodules and died within 30 days after tumor cell implantation. In the vaccinated group, only 10% (1/10) of the animals died, and 70% (7/10) of them remained totally tumor-free. Mice were euthanized when the tumor exceeded 1 cm in diameter. The interval between tumor cell injection and euthanization is used as the individual survival time. Referring to Figure 4, control mice (no vaccines were administered) and animals immunized by NIVS (10⁸ pfu of AdCMV-hcea were topically applied a month before) were subjected to tumor challenges. Numbers in parentheses represent the number of animals for each treatment. Results show that non-invasive delivery of genetic vaccines onto the skin may be able to elicit protective immune responses against tumor cells expressing a specific antigen.

### EXAMPLE 5

### Construction of recombinant adenovirus vectors encoding cytokine and co-stimulatory genes

Adenovirus vectors encoding co-stimulatory and cytokine genes were constructed for the co-delivery of these immune-modulatory genes with antigen genes into skin cells in an attempt to direct the immune profile in vaccinated animals. The adenovirus vector AdCMV-mB7.1 encoding the murine B7-1 gene and the adenovirus vector AdCMV-mgmcsf encoding the murine GM-CSF gene were constructed by homologous recombination between two transfected plasmids in human 293 cells following a standard procedure for generating new adenovirus vectors (Gomez-Foix et al., 1992). All transgenes in these vectors were transcriptionally driven by the CMV early enhancer-promoter element. AdCMV-mB7.1 was characterized by staining transduced human lung carcinoma SCC-5 cells with the anti-CD80 antibody (PharMingen), followed by flow cytometric analysis. AdCMV-mgmcsf was characterized by measuring murine GM-CSF secreted from transduced SCC-5 cells with an ELISA kit (Amersham).

### EXAMPLE 6

### Detection of antitumor immunity by in vivo cytotoxicity assay

An *in vivo* cytotoxicity assay was developed in which target cells were implanted as monolayers onto the muscle tissue of mice (Tang et al., 1996). Implantation of target cells as monolayers allowed for an efficient retrieval of target cells for assessing their fates after a few days of *in vivo* growth. This assay was particularly useful for detecting weak immune responses that are not potent enough for eradicating target cells. Immune responses can be characterized by histological analysis of the implantation bed. Without an immune response, target cells would grow. With a potent immune response, target cells would be eradicated in the presence of a large number of immune effector cells at the implantation bed, probably by virtue of migration to and *in situ* sensitization around growing target cells. With a weak immune response, growing target cells would intermingle with infiltrating immune effector cells at the implantation bed. Implanting 5 X 10⁵ RM1-luc cells [RM1 prostate tumor cells expressing the luciferase gene] as a monolayer into naive C57BL/6 mice resulted in a tumor layer due to proliferation of RM1-luc cells *in vivo,* with no evidence of immune intervention. In contrast to control animals, RM1-luc cells were intermingled with a large number of immune effector cells at the implantation bed in animals vaccinated by skin-targeted non-invasive delivery of AdCMV-luc.

### EXAMPLE 7

### Characterization of immune effector cells recruited by tumor cells

The *in vivo* cytotoxicity assay was able to concentrate a large number of immune effector cells at the implantation bed by implanting a small number of target cells as a monolayer onto muscle. Characterization of specific immune effector cells at the implantation bed may provide evidence as to whether a cell-mediated immune response has been elicited for killing target cells. For characterizing T cells that were recruited by luciferase-expressing tumor cells in animals vaccinated by skin-targeted non-invasive delivery of AdCMV-luc, tissue sections of the implantation bed were stained with an anti-CD3 monoclonal antibody (mAb). RM1-luc cells were produced by lipofecting pHBA-luc DNA into RM1 prostate tumor cells (provided by T. Thompson at the Baylor College of Medicine), followed by selection in medium containing G418. Clones expressing luciferase were characterized by luciferase assay. Five X 10⁵ RM1-luc cells were implanted as a monolayer into a mouse that had been vaccinated by skin-targeted non-invasive delivery of 10⁸ pfu AdCMV-luc. Five days after implantation, the implantation bed was frozen in O.C.T. and sections were cut at 4 µm, dried in 100% acetone, and stained with an anti-CD3 mAb (clone F500A2, provided by P. Bucy at UAB), via the ABC immunoperoxidase procedure with diaminobenzidine as the chromogen.

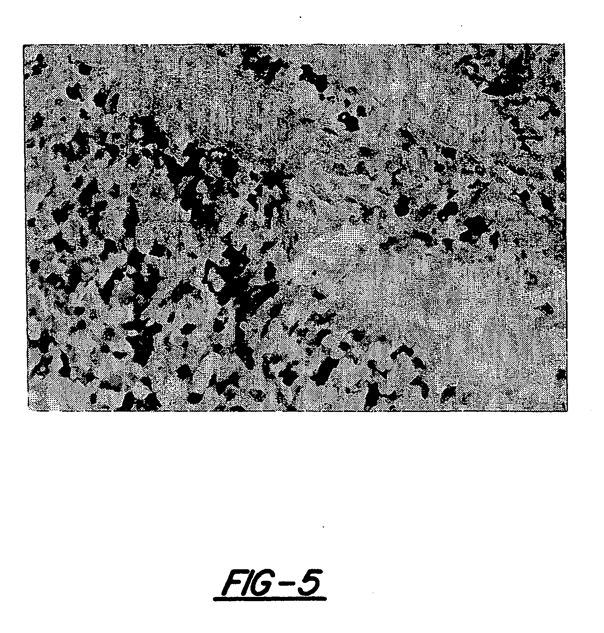

As shown in Figure 5, a large number of T cells infiltrated into the implantation bed after 5 days of *in vivo* growth of RM1-luc cells in a mouse vaccinated by skin-targeted non-invasive delivery of AdCMV-luc (x150) while only a few T cells were found in naive animals. It appeared that the same number of RM1-luc target cells could recruit more T lymphocytes to the implantation bed in vaccinated animals than in naive animals.

For characterizing CTLs that were recruited by target cells, frozen sections of the implantation bed were subjected to *in situ* hybridization using an antisense granzyme A RNA molecule as the probe. Five X 10⁵ RM1-luc cells were implanted as a monolayer into either a naive C57BL/6 mouse or a mouse that had been vaccinated by skin-targeted non-invasive delivery of 10⁸ pfu AdCMV-luc. Five days after implantation, the implantation bed was frozen in O.C.T. and sections were cut at 4 µm. Frozen sections were fixed in 3% paraformaldehyde, incubated in 0.2 M HCl for inhibiting endogenous alkaline phosphatase activity, and hybridized with a heat-denatured antisense granzyme A RNA probe. Probes for *in situ* hybridization were single-stranded RNA molecules produced by transcription from a plasmid containing bacteriophage promoters. During the transcription, digoxigenin-UTP was directly incorporated into the sequence. Sense sequence probes were used as negative controls. After hybridizing with probes, sections were washed and incubated with alkaline phosphatase-conjugated anti-digoxigenin antibody, followed by incubation in the NBT/BCIP enzyme substrate solution.

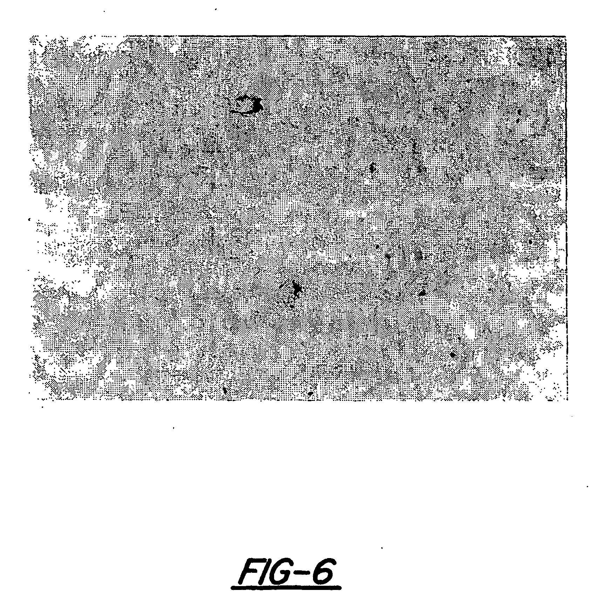

CTLs that express granzyme A are activated CTLs and have been used as predictive markers for tissue rejection during transplantation. Granzyme-positive CTLs were found within the RM1-luc implantation bed only in animals that had been vaccinated by skin-targeted non-invasive delivery of AdCMV-luc (Figure 6). Their presence at the bed suggests that a cell-mediated immune response against tumor cells expressing a specific antigen may have been induced by NIVS.

### EXAMPLE 8

### Topical application of genetic vaccines by adhesive bandages

It was demonstrated, for the first time, that bandages could be used for the administration of vaccines. This development may allow personnel without medical training to deliver a uniform dose of non-invasive vaccines onto the skin. To transduce skin by bandage, 50 µl of the AdCMV-luc vector described in Example 7 was pipetted into the pad of an adhesive bandage (Johnson & Johnson). The vector-containing bandage was subsequently adhered to pre-shaved skin of a mouse. The vector was kept in contact with naked skin for 18 hours. To detect transgene expression from genetic vectors delivered by a bandage, the skin was assayed for luciferase (Table 1). While the results show substantial variation, transgene expression in the skin was achievable using adhesive bandages.

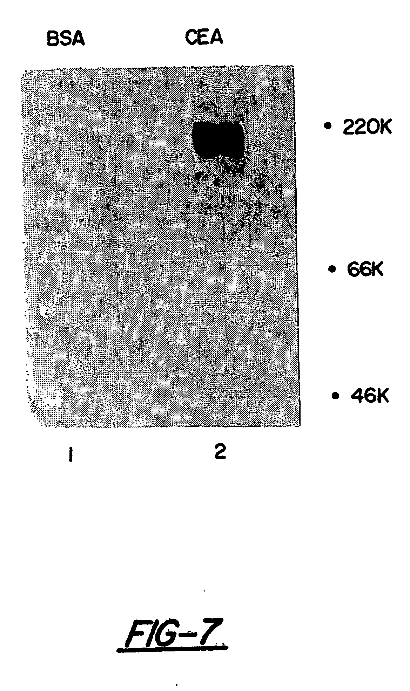

To demonstrate that animals could be vaccinated with non-invasive adhesive bandages, sera from tail bleeds were assayed for anti-CEA antibodies two months after adhering bandages containing AdCMV-hcea onto the skin of mice. As shown in Figure 7, anti-CEA antibodies were detected in 100% (10/10) of mice that received non-invasive vaccines through adhesive bandages.

### EXAMPLE 9

### DNA/adenovirus-mediated NIVS

Adenovirus-based vectors can be made more versatile by binding plasmid DNA to the exterior of an adenovirus. The resulting vector system mediates high-efficiency gene delivery to a wide variety of target cells. This approach allows greatly enhanced flexibility in terms of the size and design of foreign genes. DNA/adenovirus complexes may thus be able to deliver antigen genes into the skin via the same adenovirus receptor-mediated endocytosis pathway with more flexibility.

To demonstrate the feasibility of DNA/adenovirus-mediated NIVS, plasmid DNA encoding the human growth hormone (pCMV-GH) (Tang et al., 1992) was allowed to complex with an E4-defective adenovirus. Mice (strain C57BL/6) were vaccinated by contacting DNA/adenovirus complexes with naked skin for one day. Immunized animals were subsequently monitored for the production of antibodies against the human growth hormone protein (hGH) by assaying sera from tail-bleeds. As shown in Figure 8a, lane 1, hGH (0.5 µg); lane 2, BSA (0.5 µg), the test sera reacted in western blots with purified hGH, but not with irrelevant proteins. Often mice vaccinated by DNA/adenovirus complexes, eight (80%) produced antibodies against hGH within three months, indicating that specific antibodies could be produced against exogenous proteins encoded by plasmid DNA that is complexed with adenovirus and administered in a non-invasive mode. Pre-immune sera collected before treatment, sera from untreated animals, and sera from animals vaccinated with irrelevant vectors all failed to react with hGH. Thus, DNA/adenovirus complexes, like adenovirus recombinants, appear as a legitimate vector system for NIVS.

### EXAMPLE 10 (not illustrative of the invention)

### DNA/lipnsome-mediated NIVS

In addition to developing genetic vectors involving adenovirus as carriers for non-invasive vaccines, it has also been demonstrated that mice could be vaccinated by topical application of DNA/liposome complexes without viral elements. It is apparent that many different vectors can be applied in a creative way for the administration of skin-targeted non-invasive vaccines. As shown in Figure 8b, lane 1, hGH (0.5 µg); lane 2, BSA (0.5 µg), the test serum from a mouse immunized by topical application of DNA/liposome complexes encoding hGH reacted with hGH but not with BSA. Of 10 mice vaccinated by DNA/liposome complexes, the test sera reacted with purified hGH in 9 (90%) treated animals within 5 months. Thus, the DNA/liposome complex, like the adenovirus and the DNA/adenovirus complex, appears as another legitimate vector system for NIVS.

### EXAMPLE 11

### Co-expression of DNA-encoded and adenovirus-encoded transgenes

Strategies of augmenting the immune system's response can potentially improve the clinical outcomes of vaccines. Local production of immune-modulatory molecules involved in the activation and expansion of lymphocyte populations may significantly improve the vaccination effects. Adenovirus vectors encoding the murine B7-1 and GM-CSF genes have been made. Topical application of DNA/adenovirus complexes may thus be able to co-express DNA-encoded antigens or immune modulatory molecules with adenovirus-encoded antigens or immune modulatory molecules in individual skin cells for enhancing the immune response against the antigen.

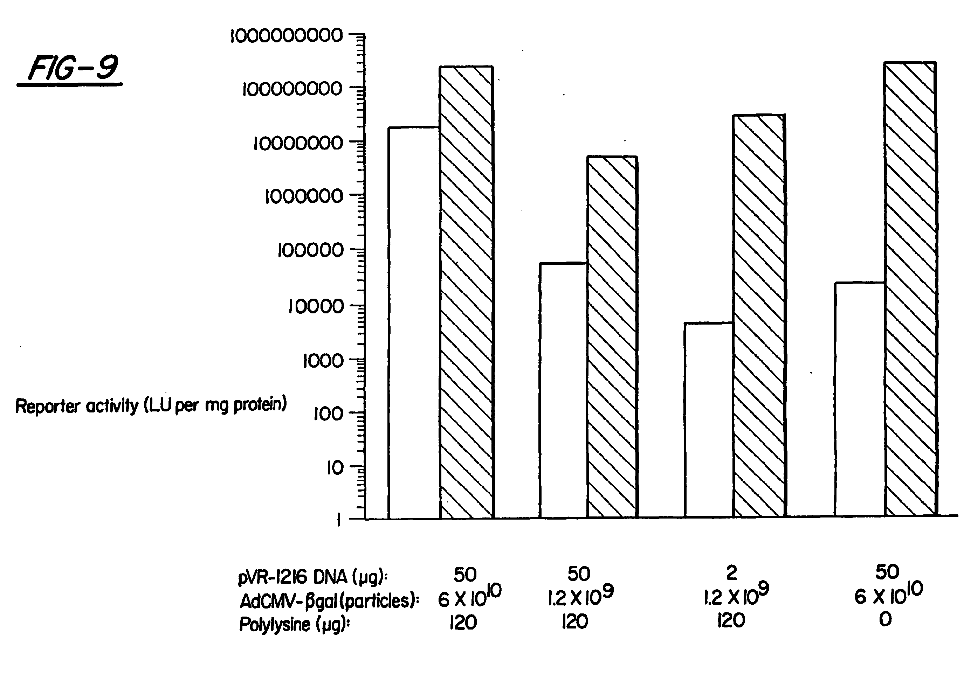

Figure 9 shows that the expression of transgenes from plasmid DNA in target cells is dependent upon the presence of adenovirus, thus allowing plasmid-encoded and adenovirus-encoded transgenes to be co-expressed in the same cell. pVR-1216 plasmid DNA (provided by Vical), AdCMV-βgal particles and polylysine were mixed at specific ratios as shown in the figure. The complex was applied to 2 X 10⁵ SCC-5 cells in a well and incubated for 2 hours. The complex was then removed and cells were harvested for luciferase and β-galactosidase assays the next day. Open column: luciferase activity; solid column: β-galactosidase activity. Results show that DNA-encoded transgenes are not expressed in target cells in the absence of adenovirus, whereas adenovirus-encoded transgenes can be expressed in the presence of DNA. It is also possible that DNA may be condensed onto the surface of other viruses for targeting different cell types. Accordingly, this protocol provides a simple but versatile gene delivery system which allows the expression of transgenes from both a virus recombinant and an externally-bound plasmid, simultaneously.

### EXAMPLE 12

### Relative transgene expression in the skin from different genetic vectors by topical application

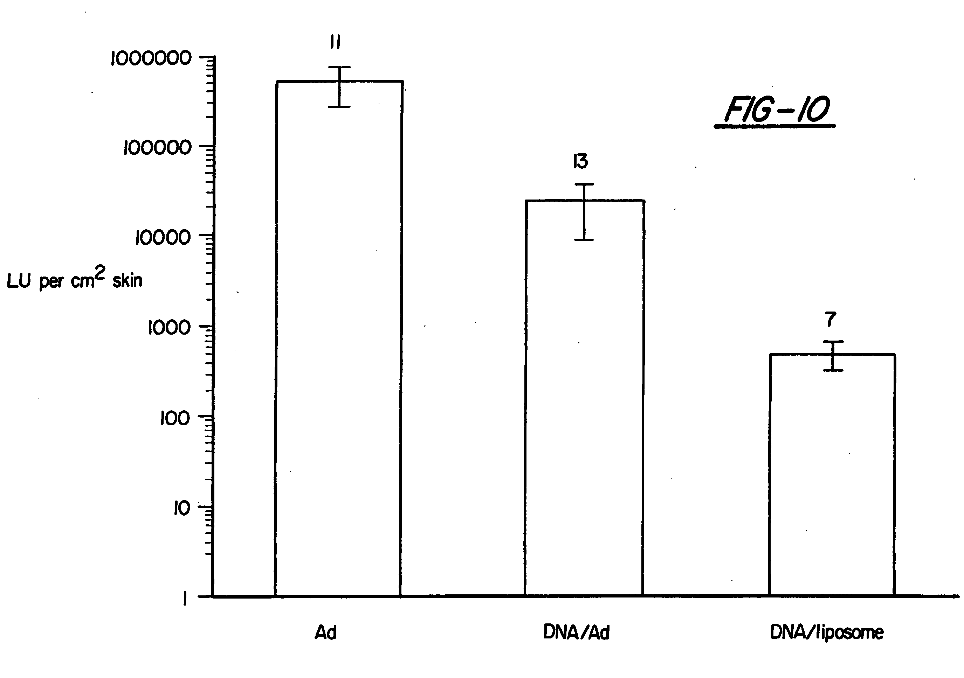

It has been shown that adenovirus recombinants, DNA/adenovirus complexes, DNA/liposome complexes, and perhaps many other genetic vectors can all be applied as carriers for non-invasive vaccines. It is conceivable that the higher the efficiency for transgene expression, the more powerful the carrier will be. To define the relative efficiencies for the vectors utilized, adenovirus recombinants, DNA/adenovirus complexes, or DNA/liposome complexes were allowed to contact mouse skin by topical application for 18 hr. The treated skin was subsequently removed from the animal and assayed for luciferase activity with a luminometer by measurement of integrated light emission for 2 min using the Promega's luciferase assay system, and background was subtracted from the readings. As shown in Figure 10, adenovirus recombinants were found to be the most efficient vector system for skin-targeted non-invasive gene delivery. Mice mock-treated produced no detectable luciferase activity in the skin. LU, light units; Ad, AdCMV-luc; DNA/Ad, pVR-1216 DNA complexed with Ad d11014; DNA/liposome, pVR-1216 DNA complexed with DOTAP/DOPE. Results are the mean log[LU per cm² skin] ± SE (n is shown on top of each column). Although the efficiency of DNA/adenovirus complex is lower than that of adenovirus recombinant, it is significantly higher than that of DNA/liposome complex. In addition, adenovirus may be inactivated by UV irradiation before complexing with DNA to prevent viable viral particles from disseminating. Thus, DNA/adenovirus complexes may appear as the most promising carrier system for the delivery of non-invasive vaccines when efficiency and safety factors are both considered in formulating a new generation of vaccines.

### REFERENCES

The following references were cited herein:
Barry, M.A. et al. Protection against mycoplasma infection using expression-library immunization. Nature 377, 632-635 (1995).
Conry, R.M. et al. A carcinoembryonic antigen polynucleotide vaccine for human clinical use. Cancer Gene Ther. 2, 33-38 (1995).
Cotten, M. et al. High-efficiency receptor-mediated delivery of small and large (48 kilobase) gene constructs using the endosome-disruption activity of defective or chemically inactivated adenovirus particles. Proc. Natl. Acad. Sci USA 89, 6094-6098 (1992).
Glenn, G.M. et al. Skin immunization made possible by cholera toxin. Nature 391, 851 (1998).
Gomez-Foix et al. Adenovirus-mediated transfer of the muscle glycogen phosphorylase gene into hepatocytes confers altered regulation of glycogen metabolism. J.Biol. Chem., 267, 25129-25134 (1992).
Johnston, S. A. & Tang, D.-c. Gene gun transfection of animal cells and genetic immunization. Meth. Cell Biol. 43, 353-365 (1994).
McDonnell, W.M. & Askari, F.K. DNA vaccines. New Engl. J. Med. 334, 42-45 (1996).
Tang, D.-c. et al. Genetic immunization is a simple method for eliciting an immune response. Nature 356, 152-154 (1992).
Tang, D.-c. et al. Butyrate-inducible and tumor-restricted gene expression by adenovirus vectors. Cancer Gene Ther. 1, 15-20 (1994).
Tang, D.-c. et al. In vivo cytotoxicity assay for assessing immunity. J. Immunol. Methods 189, 173-182 (1996).
Tang, D.-c. et al. Vaccination onto bare skin. Nature 388, 729-730 (1997).

**TABLE 1**

| **Incubation time (hours)** | **LU per cm² skin** |
|---|---|
| 1 | 0 |
| 1 | 2,100 |
| 2 | 0 |
| 2 | 0 |
| 2 | 6,200 |
| 2 | 7,300 |
| 2 | 13,000 |
| 2 | 48,000 |
| 2 | 1,800 |
| 2 | 13,000 |
| 18 | 830 |
| 18 | 2,400 |
| 18 | 260 |
| 18 | 630 |
| 18 | 1,300,000 |
| 18 | 24,000 |
| 18 | 2,700 |
| 18 | 280 |

## Claims

1. Use of an adenoviral vector encoding a transgene of interest for the preparation of a non-invasive vaccine for the induction of an immune response in an animal by non-invasive application of the vector to the skin of the animal.

2. Use according to claim 1, wherein said transgene encodes an anti tumor antigen and said immune response is an anti tumor immune response.

3. Use according to any of the preceding claims, wherein the transgene encodes an antigen.

4. Use according to any one of the preceding claims, wherein the antigen can be used against a pathogen or neoplasm.

5. Use according to any one of the preceding claims, wherein the antigen is selected from the group consisting essentially of the human carcinoembryonic antigen, the HIV gp120 antigen, and the influenza NP antigens.

6. Use according to any one of the preceding claims, wherein the vector further encodes a co-stimulatory gene and a cytokine gene.

7. Use according to any one of the preceding claims; wherein the immune modulatory gene is selected from the group consisting of a GM-CSF gene, a B7-1 gene, a B7-2 gene, an interleukin-2 gene, and an interleukin-12 gene.

8. Use according to any one of the preceding claims, wherein the adenovirus vector is defective in its E1 region.

9. Use according to any one of the preceding claims wherein the vector has all viral genes deleted.

10. Use according to any of the preceding claims, wherein the vector comprises a DNA/virus complex and the DNA is in plasmid form.

## Patentansprüche

1. Verwendung eines adenoviralen Vektors, der für ein Transgen von Interesse zur Herstellung eines non-invasiven Impfstoffes zur Induktion einer Immunreaktion bei einem Tier durch non-invasiven Auftrag des Vektors auf die Tierhaut kodiert.

2. Verwendung nach Anspruch 1, worin das Transgen für ein Antitumorantigen kodiert und die Immunreaktion eine Antitumorimmunreaktion ist.

3. Verwendung nach irgendeinem der vorgehenden Ansprüche, worin das Transgen für ein Antigen kodiert.

4. Verwendung nach irgendeinem der vorgehenden Ansprüche, worin das Antigen gegen ein Pathogen oder Neoplasma verwendet werden kann.

5. Verwendung nach irgendeinem der vorgehenden Ansprüche, worin das Antigen aus der Gruppe hauptsächlich bestehend aus dem humanen karzinoembryonales Antigen, dem HIV gp120-Antigen und den Influenza-NP-Antigenen ausgewählt ist.

6. Verwendung nach irgendeinem der vorgehenden Ansprüche, worin der Vektor weiterhin für ein co-anregendes Gen und ein Cytokingen kodiert.

7. Verwendung nach irgendeinem der vorgehenden Ansprüche, worin das immunregulierende Gen aus der Gruppe bestehend aus einem GM-CSF-Gen, einem B7-1-Gen, einem B7-2-Gen, einem Interleukin-2-Gen und einem Interleukin-12-Gen ausgewählt ist.

8. Verwendung nach irgendeinem der vorhergehenden Ansprüche, worin der Adenovirusvektor in seiner E1-Region defekt ist.

9. Verwendung nach irgendeinem der vorgehenden Ansprüche, worin alle viralen Gene des Vektors deletiert werden.

10. Verwendung nach irgendeinem der vorgehenden Ansprüche, worin der Vektor einen DNA/Viruskomplex umfasst und die DNA in plasmider Form vorliegt.

## Revendications

1. Utilisation d'un vecteur adénoviral codant pour un transgène d'intérêt dans la préparation d'un vaccin non invasif destiné à induire une réponse immunitaire chez un animal au moyen d'une application non invasive du vecteur sur la peau dudit animal.

2. Utilisation selon la revendication 1, dans laquelle ledit transgène code pour un antigène antitumoral et ladite réponse immunitaire est une réponse immunitaire antitumorale.

3. Utilisation selon l'une quelconque des revendications précédentes, dans laquelle le transgène code pour un antigène.

4. Utilisation selon l'une quelconque des revendications précédentes, dans laquelle l'antigène peut être utilisé contre un agent pathogène ou contre un néoplasme.

5. Utilisation selon l'une quelconque des revendications précédentes, dans laquelle l'antigène est choisi dans le groupe essentiellement constitué par l'antigène carcino-embryonnaire humain, l'antigène gp120 du HIV et les antigènes NP de la grippe.

6. Utilisation selon l'une quelconque des revendications précédentes, dans laquelle le vecteur code en outre pour un gène costimulateur et pour un gène de cytokine.

7. Utilisation selon l'une quelconque des revendications précédentes, dans laquelle le gène immunomodulateur est choisi dans le groupe constitué par un gène de GM-CSF, un gène de B7-1, un gène de B7-2, un gène de l'interleukine 2 et un gène de l'interleukine 12.

8. Utilisation selon l'une quelconque des revendications précédentes, dans laquelle le vecteur adénoviral est défectif dans sa région EI.

9. Utilisation selon l'une quelconque des revendications précédentes, dans laquelle le vecteur présente une délétion de tous les gènes viraux.

10. Utilisation selon l'une quelconque des revendications précédentes, dans laquelle le vecteur comprend un complexe ADN/virus et l'ADN se présente sous forme de plasmide.
